# EUROPEAN PATENT APPLICATION

(11) **EP 4 269 559 A1**
(43) Date of publication of application: **01.11.2023**
(21) Application number: 21910939.4
(22) Date of filing: 23.12.2021
(51) Int. Cl.: C12M 3/00, C08J 7/12

(54) **CELL CULTURING MEMBER, AND METHOD FOR MODIFYING SURFACE THEREOF**

(30) Priority: 25.12.2020 JP 2020216758
(71) Applicant: Shinshu University, Matsumoto-shi, Nagano 390-8621 (JP); Stella Chemifa Corporation, Osaka-shi, Osaka 541-0044 (JP)
(72) Inventor: SAITO Naoto, Matsumoto-shi, Nagano 390-8621 (JP); UEMURA Takeshi, Matsumoto-shi, Nagano 390-8621 (JP); HISANO Kazutoshi, Izumiotsu-shi, Osaka 595-0075 (JP); SATO Yoshinori, Izumiotsu-shi, Osaka 595-0075 (JP); YAMAMOTO Masashi, Izumiotsu-shi, Osaka 595-0075 (JP); NII Keiichi, Izumiotsu-shi, Osaka 595-0075 (JP); NISHIDA Tetsuo, Izumiotsu-shi, Osaka 595-0075 (JP)
(74) Representative: Meissner Bolte Partnerschaft mbB
(86) International application number: PCT/JP2021/047797
(87) International publication number: WO 2022/138799

(57) **Abstract**

Provided are a cell culture member having excellent cell culture performance and its long-term stability, and a method for modifying the surface thereof. The cell culture member according to the present invention is a cell culture member having at least a holding region that holds an adherent cell and contains a polymer compound, wherein at least a part of the holding region is a surface-modified region in which a fluorine atom is directly chemically bonded to a part of carbon atoms and/or silicon atoms constituting the polymer compound, and the present invention can provide a cell culture member that improves adhesiveness of the adherent cell to the surface-modified region, suppresses deterioration over time of adhesiveness, and is excellent in cell culture performance and its long-term stability.

## Description

### TECHNICAL FIELD

The present invention relates to a cell culture member and a method for modifying a surface thereof, and more particularly to a cell culture member having excellent cell culture performance and its long-term stability, and a method for modifying a surface thereof.

### BACKGROUND ART

The technology of cell culture is used for researches and the like in various fields such as elucidation of biochemical phenomena, production of useful substances, regenerative medicine, and drug evaluation. Among cell culture techniques, in a cell culture vessel, a polymer compound containing a thermoplastic resin is generally used as a material thereof from the viewpoints of increased recognition of infection, contamination, and the like, moldability, and production cost. The shape and physical properties of the cell culture vessel greatly affect the efficiency, analysis accuracy, and the like of researches in various applications.

In particular, in researches using adherent cells (all cells in mammals except some cells such as cancer cells and hematopoietic cells), adhesiveness between the adherent cells and the cell culture vessel is important for cell growth efficiency and physiological activity maintenance. However, most of the cell culture vessels produced using the thermoplastic resin has a hydrophobic surface, and thus has a problem that the adhesiveness of the adherent cells is poor, and the cell activity of the adherent cells is significantly inhibited.

For such a problem, for example, Patent Document 1 describes that the surface of a polypropylene substrate is modified by plasma treatment in order to deposit or immobilize cells or biomolecules on the polypropylene substrate. Patent Document 1 describes that surface modification of the polypropylene substrate is performing by subjecting the polypropylene substrate to plasma treatment, to thereby optimize interaction between the surface of the polypropylene substrate and the cells or the biomolecules.

However, when surface modification is performed by discharge treatment represented by plasma treatment, the surface of the workpiece is preferably a smooth surface without unevenness. Therefore, there is a problem that application of the discharge treatment to a cell culture vessel having a complicated shape, which is used in three-dimensional culture whose demand has increased in recent years, is difficult. In addition, the surface subjected to the discharge treatment exhibits sufficient cell culture performance immediately after the discharge treatment, but there is a problem that the cell culture performance deteriorates with the lapse of time, and thus the long-term stability is poor.

### PRIOR ART DOCUMENTS

### PATENT DOCUMENTS

Patent Document 1: JP-T-2014-515692

### SUMMARY OF THE INVENTION

### PROBLEMS TO BE SOLVED BY THE INVENTION

The present invention has been made in view of the above problems, and an object of the present invention is to provide a cell culture member having excellent cell culture performance and its long-term stability, and a method for modifying a surface thereof.

### SOLUTION TO THE PROBLEMS

In order to solve the above problems, a cell culture member according to the present invention is a cell culture member having at least a holding region that holds an adherent cell and contains a polymer compound, wherein at least a part of the holding region is a surface-modified region in which a fluorine atom is directly chemically bonded to a part of carbon atoms and/or silicon atoms constituting the polymer compound.

In the above configuration, preferably, a peak value of binding energy of the fluorine atom, as measured by X-ray photoelectron spectroscopy, is in a range of 680 eV to 690 eV.

In the above configuration, preferably, a surface-modifying group is directly chemically bonded to a part of other carbon atoms and/or other silicon atoms constituting the polymer compound in the surface-modified region, the surface-modifying group is at least one selected from a group consisting of an -OR¹ group; a -COOR² group; a -COR³ group; a hydrocarbon group; a silyl group; a hydrocarbon group having at least one of a hetero atom, a halogen atom, or an unsaturated bond; a silyl group having at least one of a hetero atom, a halogen atom, or an unsaturated bond; a cyano group; a nitro group; a nitroso group; a phosphate group; a sulfonyl group; a thiol group; a thionyl group; and a halogen atom excluding a fluorine atom, R¹ and R² are each independently a hydrogen atom; a metal atom; a hydrocarbon group; a silyl group; a hydrocarbon group having at least one of a hetero atom, a halogen atom, or an unsaturated bond; or a silyl group having at least one of a hetero atom, a halogen atom, or an unsaturated bond, and R³ is a hydrocarbon group; a hydrocarbon group having at least one of a hetero atom or an unsaturated bond; or a silyl group having at least one of a hetero atom or an unsaturated bond.

In the above configuration, preferably, the polymer compound is at least one polymer selected from a group consisting of polyvinyl chloride, polystyrene, polyethylene, polypropylene, polyvinyl acetate, polyurethane, cyclic polyolefin, polyether ether ketone, polyimide, polyamide imide, polycarbonate, polymethyl methacrylate, polyethylene terephthalate, acrylonitrile-butadiene-styrene, polyacrylonitrile, polyamide, polyvinyl alcohol, polyolefin, and a silicon-containing polymer compound.

In order to solve the above problems, a method for modifying a surface of a cell culture member of the present invention is a method for modifying a surface of a cell culture member having a holding region that holds an adherent cell and contains a polymer compound, the method including a step of bringing a first treatment gas containing: a gas containing a fluorine atom; and an inert gas as an optional component into contact with at least a part of the holding region, and forming a surface-modified region in which the fluorine atom is directly chemically bonded to a part of carbon atoms and/or silicon atoms constituting the polymer compound.

In the above configuration, preferably, a peak value of binding energy of the fluorine atom in the surface-modified region, as measured by X-ray photoelectron spectroscopy, is in a range of 680 eV to 690 eV.

In the above configuration, in the step, a surface-modifying group may be directly chemically bonded to a part of other carbon atoms and/or other silicon atoms constituting the polymer compound by using a gas further containing a gas containing an oxygen atom as the first treatment gas, the surface-modifying group may be at least one selected from a group consisting of an -OR¹ group; a -COOR² group; a -COR³ group; a hydrocarbon group; a silyl group; a hydrocarbon group having at least one of a hetero atom, a halogen atom, or an unsaturated bond; a silyl group having at least one of a hetero atom, a halogen atom, or an unsaturated bond; a nitro group; a nitroso group; a phosphate group; a sulfonyl group; a thionyl group; and a halogen atom excluding a fluorine atom, R¹ and R² may be each independently a hydrogen atom; a metal atom; a hydrocarbon group; a silyl group; a hydrocarbon group having at least one of a hetero atom, a halogen atom, or an unsaturated bond; or a silyl group having at least one of a hetero atom, a halogen atom, or an unsaturated bond, and R³ may be a hydrocarbon group; a hydrocarbon group having at least one of a hetero atom or an unsaturated bond; or a silyl group having at least one of a hetero atom or an unsaturated bond.

In the above configuration, the method for modifying a surface of a cell culture member may further include a step of bringing a second treatment gas containing another gas containing an oxygen atom into contact with at least a part of the holding region which has been brought into contact with the first treatment gas, and causing a surface-modifying group to be directly chemically bonded to a part of other carbon atoms and/or other silicon atoms constituting the polymer compound, R¹ and R² may be each independently a hydrogen atom; a metal atom; a hydrocarbon group; a silyl group; a hydrocarbon group having at least one of a hetero atom, a halogen atom, or an unsaturated bond; or a silyl group having at least one of a hetero atom, a halogen atom, or an unsaturated bond, and R³ may be a hydrocarbon group; a hydrocarbon group having at least one of a hetero atom or an unsaturated bond; or a silyl group having at least one of a hetero atom or an unsaturated bond.

In the above configuration, preferably, the method for modifying a surface of a cell culture member further includes a step of bringing the fluorine atom directly chemically bonded to the part of the carbon atoms and/or the silicon atoms and/or the surface-modifying group directly chemically bonded to the part of the other carbon atoms and/or the other silicon atoms into contact with a treatment compound that reacts with the fluorine atom and/or the surface-modifying group, and substituting the fluorine atom and/or the surface-modifying group with a surface-modifying group derived from the treatment compound.

In the above configuration, preferably, the polymer compound is at least one polymer selected from a group consisting of polyvinyl chloride, polystyrene, polyethylene, polypropylene, polyvinyl acetate, polyurethane, cyclic polyolefin, polyether ether ketone, polyimide, polyamide imide, polycarbonate, polymethyl methacrylate, polyethylene terephthalate, acrylonitrile-butadiene-styrene, polyacrylonitrile, polyamide, polyvinyl alcohol, polyolefin, and a silicon-containing polymer compound.

### EFFECTS OF THE INVENTION

According to the cell culture member of the present invention, in a holding region that holds an adherent cell and contains a polymer compound, a fluorine atom is directly chemically bonded to a part of carbon atoms and/or silicon atoms constituting the polymer compound to provide a surface-modified region, whereby the adhesiveness of the adherent cell to the surface-modified region can be improved as compared with a region not subjected to such a surface modification. As a result, a cell culture member having excellent cell culture performance can be provided. In addition, the fluorine atom is directly chemically bonded and fixed to a part of carbon atoms or the like constituting the polymer compound.

Thus, the cell culture member of the present invention can suppress deterioration over time of adhesiveness of the adherent cell as compared with, for example, conventional cell culture members subjected to surface modification such as plasma treatment. As a result, a cell culture member having excellent long-term stability of cell culture performance can be provided.

In addition, according to the method for modifying the surface of a cell culture member of the present invention, by only bringing a first treatment gas containing at least a gas containing a fluorine atom into contact with at least a part of the holding region of the cell culture member, a fluorine atom can be directly chemically bonded to a part of carbon atoms or the like in a polymer compound constituting the holding region, so that a surface-modified region can be formed. As a result, a cell culture member having excellent cell culture performance and its long-term stability can be produced by an extremely simple method.

### BRIEF DESCRIPTION OF THE DRAWINGS

FIG. 1 is a conceptual view for explaining a surface-modified region in a cell culture member according to an embodiment of the present invention.
FIG. 2 is a conceptual view for explaining a method for modifying the surface of the cell culture member according to the embodiment.
FIG. 3 is a graph showing a measurement result of XPS of a microplate according to Example 1.
FIG. 4 is a view showing a bright field image observed with an inverted microscope after mouse astrocyte cells are cultured using a microplate of Example 1.
FIG. 5 is a view showing a bright field image observed with an inverted microscope after mouse astrocyte cells are cultured using a microplate of Comparative Example 1.
FIG. 6 is a view showing a bright field image observed with an inverted microscope after mouse astrocyte cells are cultured using a microplate of Comparative Example 2.

### EMBODIMENTS OF THE INVENTION

### (Cell culture member)

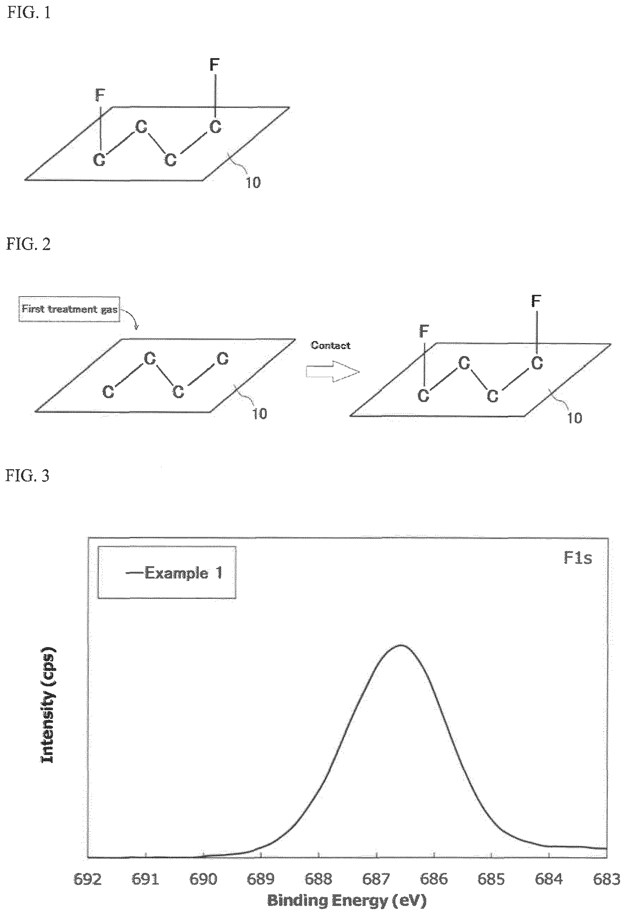

First, a cell culture member of the present embodiment will be described with reference to FIG. 1. FIG. 1 is a conceptual view for explaining a surface-modified region in a cell culture member 10 according to the present embodiment.

The cell culture member 10 of the present embodiment includes at least a holding region (cell culture surface) capable of holding adherent cells. The cell culture member 10 of the present embodiment allows the adherent cells to adhere to the holding region, and thus enables culturing of the adherent cells in the holding region.

In the present specification, the term "cell culture member" means a member having at least a solid surface that can serve as a scaffold for adherent cells in proliferation, differentiation, survival, and the like of the adherent cells. Therefore, as the cell culture member 10, for example, a film (membrane), a sheet, a microplate, a flask, a dish, a tube, a hollow fiber membrane, a substantially spherical material, or the like is used. Note that an aspect in which the cell culture member 10 is a film (membrane) or a sheet includes, for example, a case where the cell culture member 10 is a component of a finished product of a cell culture vessel or the like.

In the present specification, the term "adherent cell" means a cell that requires a scaffold on a solid surface and adheres to the solid surface in proliferation, differentiation, survival, and the like thereof. Examples of the adherent cell include epithelial cells such as human embryonic kidney cells (HEK293T cells), Syrian hamster kidney cells (BHK-21 (C-13) cells), and mouse cerebral cortex nerve cells, and glial cells (neuroglial cells) such as tumor cells, endothelial cells, fibroblasts, muscle cells, nerve/endocrine cells, primary cultured cells, and mouse astrocyte cells. However, the adherent cell is not limited to these exemplified cells.

In the cell culture member 10, at least the holding region (cell culture surface) is only required to contain a polymer compound. The holding region contains a polymer compound, and further, may be a surface-modified surface whose surface is modified by a known surface treatment such as plasma treatment.

The polymer compound is at least one polymer selected from the group consisting of polyvinyl chloride, polystyrene, polyethylene, polypropylene, polyvinyl acetate, polyurethane, cyclic polyolefin, polyether ether ketone, polyimide, polyamide imide, polycarbonate, polymethyl methacrylate, polyethylene terephthalate, acrylonitrile-butadiene-styrene, polyacrylonitrile, polyamide, polyvinyl alcohol, polyolefin, and a silicon-containing polymer compound. The polymer includes not only a thermoplastic resin but also an elastomer. Among these polymer compounds, polystyrene, polyethylene terephthalate, and polypropylene are preferable from the viewpoint of moldability, production cost, and the like.

At least a part of the holding region is a surface-modified region in which a fluorine atom is directly chemically bonded to a part of carbon atoms and/or silicon atoms (hereinafter, referred to as "carbon atoms or the like") constituting the polymer compound (see FIG. 1). As a result, in the surface-modified region to which a fluorine atom is directly chemically bonded, the adhesiveness of the adherent cell is increased as compared with a region to which a fluorine atom is not directly chemically bonded, and the cell culture performance can be improved. Further, the fluorine atom is directly chemically bonded to a part of the carbon atoms or the like of the polymer compound forming the holding region. It is therefore possible to suppress deterioration over time of the adhesiveness of the adherent cell in the surface-modified region, as compared with a region subjected to another surface treatment such as plasma treatment, for example. As a result, the long-term stability of cell culture performance can also be improved.

In the present invention, the surface-modified region is only required to be formed in at least a part of the holding region. Therefore, in the present invention, the entire region of the holding region may be the surface-modified region.

The peak value of the binding energy of the fluorine atom in the surface-modified region, as measured by X-ray photoelectron spectroscopy (XPS), is preferably in a range of 680 eV to 690 eV, more preferably in a range of 682 eV to 690 eV, and particularly preferably in a range of 683 eV to 690 eV. By setting the peak value of the binding energy to 680 eV or more, the surface-modified region is hydrophilized, and the adhesion performance can be increased. By setting the peak value of the binding energy to 690 eV or less, hydrophobization due to excessive surface modification can be prevented, and appropriate adhesiveness between the surface-modified region and the adherent cell can be maintained. The peak value of the binding energy of the fluorine atom can be measured with, for example, an X-ray photoelectron spectrometer (model number: PHI VersaProbe III, manufactured by ULVAC-PHI, Inc.) using an AlKα ray monochromatized with a monochromator as an X-ray to be emitted, under the conditions of an X-ray output of 50 W, an acceleration voltage of 15 kV, a measurement region having a diameter of about 200 µmin one measurement, and a measurement interval of binding energy of 0.05 eV.

A surface-modifying group may be further directly chemically bonded to a part of other carbon atoms and/or other silicon atoms (hereinafter, referred to as "other carbon atoms or the like") constituting the polymer compound of the surface-modified region.

The surface-modifying group is at least one selected from the group consisting of an -OR¹ group; a -COOR² group; a -COR³ group; a hydrocarbon group; a silyl group; a hydrocarbon group having at least one of a hetero atom, a halogen atom, or an unsaturated bond (hereinafter, referred to as "hydrocarbon group having a hetero atom or the like"); a silyl group having at least one of a hetero atom, a halogen atom, or an unsaturated bond (hereinafter, referred to as "silyl group having a hetero atom or the like"); a cyano group; a nitro group; a nitroso group; a phosphate group; a sulfonyl group; a thiol group; a thionyl group; and a halogen atom excluding a fluorine atom.

R¹ of the -OR¹ group and R² of the -COOR² group in the surface-modifying group are each independently a hydrogen atom; a metal atom; a hydrocarbon group; a silyl group; a hydrocarbon group having at least one of a hetero atom, a halogen atom, or an unsaturated bond; or a silyl group having at least one of a hetero atom, a halogen atom, or an unsaturated bond.

The metal atom in R¹ and R² is not particularly limited, and examples thereof include alkali metals such as lithium, sodium, and potassium; alkaline earth metals such as beryllium, magnesium, and calcium; Group 13 elements in the periodic table, such as aluminum, gallium, and indium; and lanthanoids such as lanthanum.

The hydrocarbon group in R¹ and R² is not particularly limited, and examples thereof include chain hydrocarbon groups having 1 to 100, preferably 1 to 50, more preferably 1 to 20 carbon atoms; and cyclic hydrocarbon groups having 3 to 30, preferably 3 to 20, and more preferably 3 to 12 carbon atoms. More specific examples of the chain hydrocarbon group include linear hydrocarbon groups such as a methyl group, an ethyl group, a n-propyl group, a n-butyl group, a n-pentyl group, a n-hexyl group, a n-heptyl group, and a n-octyl group; and branched hydrocarbon groups such as an isopropyl group, an isobutyl group, a tert-butyl group, and an isopentyl group. More specific examples of the cyclic hydrocarbon group include a cyclopentyl group and a cyclohexyl group. In the present specification, when the range of the number of carbon atoms is represented, it means that the range includes the number of carbon atoms of all integers included in the range. Therefore, for example, a hydrocarbon group having "1 to 3 carbon atoms" means all hydrocarbon groups having 1, 2, and 3 carbon atoms.

The silyl group in R¹ and R² is not particularly limited, and examples thereof include a trimethylsilyl group, a triethylsilyl group, a tert-butyldimethylsilyl group, a triisopropyl silyl group, and a tert-butyldiphenylsilyl (TBDPS) group.

In the hydrocarbon group having a hetero atom or the like in R¹ and R², the hetero atom means an oxygen atom, a nitrogen atom, a sulfur atom, or the like, and the halogen atom means a fluorine atom, a chlorine atom, a bromine atom, or an iodine atom. The hydrocarbon group having a hetero atom means a hydrocarbon group in which a part or all of hydrogens and carbons in the hydrocarbon group is substituted with any of these hetero atoms. In addition, the hydrocarbon group having a halogen atom means a group in which a part or all of hydrogens and carbons in the hydrocarbon group is substituted with any of these halogen atoms.

The number of carbon atoms of the hydrocarbon group having a hetero atom or the like in R¹ and R² is 1 to 100, preferably 1 to 50, and more preferably 1 to 20. In addition, the number of unsaturated bonds in the hydrocarbon group having a hetero atom or the like in R¹ and R² can be appropriately set as necessary.

More specific examples of the hydrocarbon group having a hetero atom or the like in R¹ and R² include a 2-methoxyethyl group, and the like.

In the silyl group having a hetero atom or the like in R¹ and R², the hetero atom and the halogen atom are the same as the hetero atom and the halogen atom in the hydrocarbon group having a hetero atom or the like in R¹ and R². Therefore, a detailed description thereof will be omitted.

The silyl group having a hetero atom or the like in R¹ and R² is not particularly limited, and examples thereof include a 2-methoxysilyl group, and the like. The number of unsaturated bonds in the silyl group having a hetero atom or the like in R¹ and R² can be appropriately set as necessary.

R³ of the -COR³ group in the surface-modifying group is a hydrocarbon group; a hydrocarbon group having at least one of a hetero atom or an unsaturated bond (hereinafter, referred to as "hydrocarbon group having a hetero atom or the like"); or a silyl group having at least one of a hetero atom or an unsaturated bond (hereinafter, referred to as "silyl group having a hetero atom or the like").

The hydrocarbon group in R³ is not particularly limited, and examples thereof include chain hydrocarbon groups having 1 to 100, preferably 1 to 50, more preferably 1 to 20 carbon atoms; and cyclic hydrocarbon groups having 3 to 30, preferably 3 to 20, and more preferably 3 to 12 carbon atoms. More specific examples of the chain hydrocarbon group include linear hydrocarbon groups such as a methyl group, an ethyl group, a n-propyl group, a n-butyl group, a n-pentyl group, a n-hexyl group, a n-heptyl group, and a n-octyl group; and branched hydrocarbon groups such as an isopropyl group, an isobutyl group, a tert-butyl group, and an isopentyl group. More specific examples of the cyclic hydrocarbon group include a cyclopentyl group and a cyclohexyl group.

In the hydrocarbon group having a hetero atom or the like in R³ and the silyl group having a hetero atom or the like in R³, the hetero atom is the same as the hetero atom in the hydrocarbon group having a hetero atom or the like in R¹ and R². Therefore, a detailed description thereof will be omitted.

The hydrocarbon group having a hetero atom or the like in R³ is not particularly limited, and examples thereof include a 2-methoxyethyl group, and the like. The number of unsaturated bonds in the hydrocarbon group having a hetero atom or the like in R³ can be appropriately set as necessary.

The silyl group having a hetero atom or the like in R³ is not particularly limited, and examples thereof include a 2-methoxysilyl group, and the like. The number of unsaturated bonds in the silyl group having a hetero atom or the like in R³ can be appropriately set as necessary.

The hydrocarbon group in the surface-modifying group is not particularly limited, and examples thereof include chain hydrocarbon groups having 1 to 100, preferably 1 to 50, more preferably 1 to 20 carbon atoms; and cyclic hydrocarbon groups having 3 to 30, preferably 3 to 20, and more preferably 3 to 12 carbon atoms. More specific examples of the chain hydrocarbon group include linear hydrocarbon groups such as a methyl group, an ethyl group, a n-propyl group, a n-butyl group, a n-pentyl group, a n-hexyl group, a n-heptyl group, and a n-octyl group; and branched hydrocarbon groups such as an isopropyl group, an isobutyl group, a tert-butyl group, and an isopentyl group. More specific examples of the cyclic hydrocarbon group include a cyclopentyl group and a cyclohexyl group.

The silyl group in the surface-modifying group is not particularly limited, and examples thereof include a trimethylsilyl group, a triethylsilyl group, a tert-butyldimethylsilyl group, a triisopropyl silyl group, and a tert-butyldiphenylsilyl (TBDPS) group.

The number of carbon atoms of the hydrocarbon group having a hetero atom or the like in the surface-modifying group is 1 to 100, preferably 1 to 50, and more preferably 1 to 20. In addition, the number of unsaturated bonds of the hydrocarbon group having a hetero atom or the like in the surface-modifying group can be appropriately set as necessary.

More specific examples of the hydrocarbon group having a hetero atom or the like in the surface-modifying group include a 2-methoxyethyl group, and the like.

In the hydrocarbon group having a hetero atom or the like in the surface-modifying group, the hetero atom and the halogen atom are the same as the hetero atom and the halogen atom in the hydrocarbon group having a hetero atom or the like in R¹ and R². Therefore, a detailed description thereof will be omitted.

In the silyl group having a hetero atom or the like in the surface-modifying group, the hetero atom and the halogen atom are the same as the hetero atom and the halogen atom in the hydrocarbon group having a hetero atom or the like in R¹ and R². Therefore, a detailed description thereof will be omitted.

The silyl group having a hetero atom or the like in the surface-modifying group is not particularly limited, and examples thereof include a 2-methoxysilyl group, and the like. The number of unsaturated bonds in the silyl group having a hetero atom or the like in the surface-modifying group can be appropriately set as necessary.

The sulfonyl group in the surface-modifying group is not particularly limited, and examples thereof include a mesyl group, a tosyl group, a nosyl group, and a trifluoromethanesulfonyl group.

The thionyl group in the surface-modifying group is not particularly limited, and examples thereof include a thionyl chloride group and a thionyl fluoride group.

As described above, the cell culture member 10 of the present embodiment has a structure in which a fluorine atom or a surface-modifying group such as a -OH group or a -COOH group is directly chemically bonded in the holding region that holds the adherent cell. Therefore, the cell culture member 10 of the present embodiment can sufficiently enhance the adhesiveness to the adherent cell, and is excellent in cell culture performance. The cell culture member 10 of the present embodiment is also excellent in long-term stability of excellent culture performance.

### (Method for modifying surface of cell culture member)

Next, a method for modifying the surface of the cell culture member of the present embodiment will be described with reference to FIG. 2.

The method for modifying the surface of the cell culture member of the present embodiment includes at least a step of bringing a first treatment gas into contact with at least a part of the holding region of the cell culture member 10, to perform a surface modification treatment, thus forming a surface-modified region.

The first treatment gas contains a gas containing a fluorine atom, and an inert gas as an optional component. By bringing the first treatment gas into contact with at least a part of the holding region, a fluorine atom is directly chemically bonded to a part of carbon atoms or the like constituting the polymer compound in the holding region, and the fluorination treatment can be performed. As a result, a fluorinated surface-modified region can be formed in a region of the holding region which has been in contact with the first treatment gas. The surface modification treatment of the present embodiment allows a fluorine atom to be directly chemically bonded to a part of carbon atoms or the like constituting the polymer compound. Such a treatment enables, for example, a surface treatment excellent in long-term stability, unlike plasma treatment in which the surface is activated using plasma to impart hydrophilicity.

Examples of the method for bringing the first treatment gas into contact with the holding region include a method of bringing the first treatment gas into contact with the holding region in a gas phase.

The concentration of the gas containing a fluorine atom in the first treatment gas is preferably in a range of 0.01 to 60 vol%, more preferably in a range of 0.05 to 30 vol%, and particularly preferably in a range of 0.1 to 20 vol% with respect to the total volume of the first treatment gas. By setting the concentration of the gas containing a fluorine atom to 0.01 vol% or more, it is possible to prevent fluorination of the holding region from being insufficient. In addition, by setting the concentration of the gas containing a fluorine atom to 60 vol% or less, it is possible to prevent the polymer compound in the holding region and the fluorine atom from vigorously reacting and burning at the time of treatment.

The gas containing a fluorine atom is not particularly limited as long as it is a gas containing a fluorine atom. Examples of the gas containing a fluorine atom include hydrogen fluoride (HF), fluorine (F₂), chlorine trifluoride (ClF₃), sulfur tetrafluoride (SF₄), boron trifluoride (BF₃), nitrogen trifluoride (NF₃), carbonyl fluoride (COF₂), and phosphorus pentafluoride (PF₅). These may be used singly or in combination of two or more thereof.

The first treatment gas may also contain an inert gas. The inert gas is not particularly limited, but a gas that reacts with the gas containing a fluorine atom to adversely affect the surface modification treatment of the holding region, a gas that reacts with the polymer compound to adversely affect the treatment, and a gas containing impurities that adversely affect the treatment are not preferable. Specific examples of the inert gas include nitrogen, argon, helium, neon, krypton, and xenon. These can be used singly or in combination of two or more thereof. In addition, the purity of the inert gas is not particularly limited, but the concentration of impurities adversely affecting the treatment is preferably 100 ppm or less, more preferably 10 ppm or less, and particularly preferably 1 ppm or less.

The first treatment gas may also contain a gas containing an oxygen atom. Thus, in the surface modification treatment of the present embodiment, it is also possible to introduce a surface-modifying group in addition to the fluorination treatment. That is, by containing the gas containing an oxygen atom in the first treatment gas, the surface-modifying group can be further directly chemically bonded to a part of other carbon atoms or the like constituting the polymer compound in the holding region.

The gas containing an oxygen atom is not particularly limited, but a gas that reacts with the gas containing an oxygen atom to adversely affect the surface modification treatment (fluorination treatment) of the cell culture member 10, a gas that reacts with the material or the like constituting the cell culture member 10 to adversely affect the treatment, and a gas containing impurities that adversely affect the treatment are not preferable. Specific examples of the gas containing an oxygen atom include oxygen, ozone, water vapor, carbon monoxide, carbon dioxide, phosgene, and sulfur dioxide. These can be used singly or in combination of two or more thereof.

The treatment temperature at the time of performing the surface modification treatment is not particularly limited as long as it is equal to or lower than the glass transition point of the polymer compound constituting the cell culture member 10, and is preferably -20 °C to 150 °C, more preferably -10 °C to 120 °C, and still more preferably 0 °C to 100 °C. By setting the treatment temperature to -20 °C or higher, the surface modification treatment, particularly the fluorination treatment can be promoted.

On the other hand, by setting the treatment temperature to 150 °C or lower, it is possible to suppress an excessive increase in defects of the carbon skeleton and/or the silicon skeleton generated along with introduction of fluorine atoms (fluorine groups) into the surface of the cell culture member 10, and to prevent excessive destruction of the carbon skeleton and/or the silicon skeleton and decrease in mechanical strength of the cell culture member 10. Further, it is possible to prevent the occurrence of thermal deformation in the cell culture member 10, and to suppress a decrease in yield.

The treatment time (reaction time) of the surface modification treatment is preferably in a range of 1 second to 24 hours, more preferably in a range of 1 minute to 12 hours, and particularly preferably in a range of 3 minutes to 1 hour. By setting the treatment time to 1 second or more, the surface modification, particularly fluorination of the surface of the cell culture member 10 can be made sufficient. On the other hand, by setting the treatment time to 24 hours or less, it is possible to prevent a decrease in treatment efficiency due to an increase in treatment time.

The pressure condition for performing the surface modification treatment is not particularly limited, and the treatment can be performed under normal pressure, under pressure, or under reduced pressure. The treatment is preferably performed under normal pressure from the viewpoint of economy and safety. The term "normal pressure" means standard atmospheric pressure (101.3 kPa), but the normal pressure in the present invention may also include a case of being under a pressure condition of ± 10 % with respect to the standard atmospheric pressure.

The reaction vessel for performing the surface modification treatment is not particularly limited, and conventionally known reaction vessels such as a fixed bed reaction vessel or a fluidized bed reaction vessel can be adopted.

The method for bringing the first treatment gas into contact with the cell culture member 10 is not particularly limited, and examples thereof include a method in which the first treatment gas is brought into contact with the cell culture member 10 in a sealed state under a flow of the first treatment gas or under an atmosphere containing at least the first treatment gas. The surface modification treatment may be performed a plurality of times. As a result, a fluorine atom or a surface-modifying group can be further introduced into the surface of the cell culture member 10, so that the long-term stability of the surface of the cell culture member 10 can be further improved.

The surface modification treatment on the cell culture member 10 may be applied to at least an optional partial region of the holding region that holds the adherent cell. Therefore, the surface modification treatment may be performed on the entire surface of the holding region. The holding region may also be subjected to another known surface treatment in advance before being subjected to the surface modification treatment. Examples of the known surface treatment include discharge treatment such as plasma treatment. The present invention may also include a case where a known surface treatment is applied to a region other than the holding region.

When the surface modification treatment is performed on an optional partial region (partial surface modification treatment), the surface modification treatment can be performed by masking a region other than a region where the surface modification treatment is to be performed. The masking material used for masking is not particularly limited except that the masking material has heat resistance to the treatment temperature at the time of the surface modification treatment. Specific examples of the masking material include materials containing fluororesins such as polytetrafluoroethylene, polytetrafluorochloroethylene, polyvinyl fluoride, polyvinylidene fluoride, polydichlorodifluoroethylene, and polytrifluorochloroethylene, ceramics, polyimide, polyether ether ketone (PEEK), and metals.

A post-treatment step may be performed immediately after the surface modification treatment. The post-treatment step is a step of replacing the first treatment gas with an inert gas to bring the atmosphere into an inert atmosphere, and cooling the cell culture member 10 to room temperature. The cooling to room temperature may be performed by air cooling. In addition, after evacuation for replacement with an inert gas, the pressure may be brought into atmospheric pressure with an inert gas.

This makes it possible to prevent the fluorine gas from being physically adsorbed and remaining on the surface of the cell culture member 10 subjected to the surface modification treatment. As a result, generation of hydrogen fluoride as by-products resulting from hydrolysis of fluorine gas is prevented, and defects such as disruption of cultured cells by hydrogen fluoride do not occur. The inert gas is not particularly limited, and examples thereof include nitrogen gas, and the like.

In addition, after the surface modification treatment, a second treatment gas containing another gas containing an oxygen atom may be brought into contact with the surface-modified region, to introduce the above surface-modifying group (first surface-modifying group introduction treatment).

Specific examples of another gas containing an oxygen atom include oxygen, ozone, water vapor, carbon monoxide, carbon dioxide, phosgene, and sulfur dioxide. These can be used singly or in combination of two or more thereof.

The second treatment gas may also contain an inert gas. The inert gas is not particularly limited, but a gas that reacts with the gas containing an oxygen atom to adversely affect the surface modification treatment of the holding region, a gas that reacts with the polymer compound to adversely affect the treatment, and a gas containing impurities that adversely affect the treatment are not preferable. Specific examples of the inert gas include nitrogen, argon, helium, neon, krypton, and xenon. These can be used singly or in combination of two or more thereof. In addition, the purity of the inert gas is not particularly limited, but the concentration of impurities adversely affecting the treatment is preferably 100 ppm or less, more preferably 10 ppm or less, and particularly preferably 1 ppm or less.

The treatment temperature at the time of performing the first surface-modifying group introduction treatment is not particularly limited as long as it is equal to or lower than the glass transition point of the polymer compound constituting the cell culture member 10, and is preferably -20 °C to 150 °C, more preferably -10 °C to 120 °C, and still more preferably 0 °C to 100 °C. By setting the treatment temperature to -20 °C or higher, introduction of the surface-modifying group can be promoted.

On the other hand, by setting the treatment temperature to 150 °C or lower, it is possible to suppress an excessive increase in defects of the carbon skeleton and/or the silicon skeleton generated along with the treatment of the surface of the cell culture member 10 using the second treatment gas, and to prevent excessive destruction of the carbon skeleton and/or the silicon skeleton and a decrease in mechanical strength of the cell culture member 10. Further, it is possible to prevent the occurrence of thermal deformation in the cell culture member 10, and to suppress a decrease in yield.

The treatment time (reaction time) at the time of performing the first surface-modifying group introduction treatment is preferably in a range of 1 second to 24 hours, more preferably in a range of 1 minute to 12 hours, and particularly preferably in a range of 3 minutes to 1 hour. By setting the treatment time to 1 second or more, introduction of the surface-modifying group can be made sufficient. On the other hand, by setting the treatment time to 24 hours or less, it is possible to prevent a decrease in treatment efficiency due to an increase in treatment time.

The pressure condition for performing the first surface-modifying group introduction treatment is not particularly limited, and the treatment can be performed under normal pressure, under pressure, or under reduced pressure. The treatment is preferably performed under normal pressure from the viewpoint of economy and safety.

The reaction vessel for performing the first surface-modifying group introduction treatment is not particularly limited, and conventionally known reaction vessels such as a fixed bed reaction vessel or a fluidized bed reaction vessel can be adopted.

The method for bringing the second treatment gas into contact with the cell culture member 10 is not particularly limited, and examples thereof include a method in which the second treatment gas is brought into contact with the cell culture member 10 in a sealed state under a flow of the second treatment gas or under an atmosphere containing at least the second treatment gas. The first surface-modifying group introduction treatment may be performed a plurality of times. As a result, a hydrophilic group such as a -OH group or a -COOH group can be further introduced into the surface of the cell culture member 10, and the long-term stability of the surface of the cell culture member 10 can be further improved.

The first surface-modifying group introduction treatment may be applied to at least an optional partial region of the holding region that holds the adherent cell. Therefore, the first surface-modifying group introduction treatment may be performed on the entire surface of the holding region. When the first surface-modifying group introduction treatment is performed on an optional partial region (partial surface-modifying group introduction treatment), the first surface-modifying group introduction treatment can be performed by masking a region other than a region where the first surface-modifying group introduction treatment is to be performed. The masking material used for masking is not particularly limited except that the masking material has heat resistance to the treatment temperature at the time of the first surface-modifying group introduction treatment. Specific examples of the masking material include the above masking materials used when the partial surface modification treatment is performed.

In addition, immediately after the first surface-modifying group introduction treatment, another surface-modifying group may be introduced into the surface-modified region (second surface-modifying group introduction treatment). The second surface-modifying group introduction treatment is performed by bringing a treatment compound that reacts with a fluorine atom directly chemically bonded to a part of carbon atoms or the like and/or a surface-modifying group directly chemically bonded to a part of other carbon atoms or the like, into contact with the fluorine atom and/or the surface-modifying group. Thus, in the second surface-modifying group introduction treatment, the fluorine atom and/or the surface-modifying group can be substituted with a surface-modifying group derived from the compound.

The surface-modifying group derived from the treatment compound is the same as the surface-modifying group in the first surface-modifying group introduction treatment. Therefore, a detailed description thereof will be omitted.

As the treatment compound, a gaseous, liquid, or solid compound can be used without any particular limitation as long as the compound reacts with a fluorine atom and/or a surface-modifying group.

The gaseous treatment compound is not particularly limited, and examples thereof include water vapor, and the like. The gaseous treatment compound may contain an inert gas. The inert gas is not particularly limited, but a gas that reacts with the treatment compound to adversely affect the surface modification treatment of the holding region, a gas that reacts with the polymer compound to adversely affect the treatment, and a gas containing impurities that adversely affect the treatment are not preferable.

Specific examples of the inert gas include nitrogen, argon, helium, neon, krypton, and xenon. These can be used singly or in combination of two or more thereof. In addition, the purity of the inert gas is not particularly limited, but the concentration of impurities adversely affecting the treatment is preferably 100 ppm or less, more preferably 10 ppm or less, and particularly preferably 1 ppm or less.

The liquid treatment compound is not particularly limited, and examples thereof include water, and the like. When the treatment compound is water, the treatment can also be performed as a cleaning treatment of the cell culture member 10 described later. Details of the cleaning treatment will be described later.

The treatment temperature at the time of performing the second surface-modifying group introduction treatment is not particularly limited as long as it is equal to or lower than the glass transition point of the polymer compound constituting the cell culture member 10, and is preferably -20 °C to 150 °C, more preferably -10 °C to 120 °C, and still more preferably 0 °C to 100 °C. By setting the treatment temperature to -20 °C or higher, introduction of the surface-modifying group derived from the treatment compound can be promoted.

On the other hand, by setting the treatment temperature to 150 °C or lower, it is possible to suppress an excessive increase in defects in the carbon skeleton and/or the silicon skeleton generated along with the treatment of the surface of the cell culture member 10 using the treatment compound, and to prevent excessive destruction of the carbon skeleton and/or the silicon skeleton and a decrease in mechanical strength of the cell culture member 10. Further, it is possible to prevent the occurrence of thermal deformation in the cell culture member 10, and to suppress a decrease in yield.

The treatment time (reaction time) at the time of performing the second surface-modifying group introduction treatment is preferably in a range of 1 second to 24 hours, more preferably in a range of 1 minute to 12 hours, and particularly preferably in a range of 3 minutes to 1 hour. By setting the treatment time to 1 second or more, introduction of the surface-modifying group derived from the treatment compound can be made sufficient. On the other hand, by setting the treatment time to 24 hours or less, it is possible to prevent a decrease in treatment efficiency due to an increase in treatment time.

The pressure condition for performing the second surface-modifying group introduction treatment is not particularly limited, and the treatment can be performed under normal pressure, under pressure, or under reduced pressure. The treatment is preferably performed under normal pressure from the viewpoint of economy and safety.

The reaction vessel for performing the second surface-modifying group introduction treatment is not particularly limited, and conventionally known reaction vessels such as a fixed bed reaction vessel or a fluidized bed reaction vessel can be adopted.

When the treatment compound is a gaseous compound, the method for bringing the treatment compound into contact with the cell culture member 10 is not particularly limited, and examples thereof include a method in which the treatment compound is brought into contact with the cell culture member 10 in a sealed state under a flow of the treatment compound or under an atmosphere containing at least the treatment compound. The second surface-modifying group introduction treatment may be performed a plurality of times.

The second surface-modifying group introduction treatment may be applied to at least an optional partial region of the holding region that holds the adherent cell. Therefore, the second surface-modifying group introduction treatment may be performed on the entire surface of the holding region. When the second surface-modifying group introduction treatment is performed on an optional partial region (partial surface-modifying group introduction treatment), the second surface-modifying group introduction treatment can be performed by masking a region other than a region where the second surface-modifying group introduction treatment is to be performed.

The masking material used for masking is not particularly limited except that the masking material has heat resistance to the treatment temperature at the time of the second surface-modifying group introduction treatment. Specific examples of the masking material include the above masking materials used when the first surface-modifying group introduction treatment is performed.

In the present embodiment, the post-treatment may be performed immediately after the first surface-modifying group introduction treatment and the second surface-modifying group introduction treatment. The post-treatment is a step of replacing the second treatment gas or the gaseous treatment compound with an inert gas to bring the atmosphere into an inert atmosphere, and cooling the cell culture member 10 to room temperature. The cooling to room temperature may be performed by air cooling. In addition, after evacuation for replacement with an inert gas, the pressure may be brought into atmospheric pressure with an inert gas.

Thus, when the post-treatment step is performed immediately after the first surface-modifying group introduction treatment, it is possible to prevent the second treatment gas from being adsorbed and remaining on the surface of the cell culture member 10 subjected to the first surface-modifying group introduction treatment. When the post-treatment step is performed immediately after the second surface-modifying group introduction treatment, it is possible to prevent the treatment compound from being adsorbed and remaining on the surface of the cell culture member 10 subjected to the second surface-modifying group introduction treatment.

As a result, generation of by-products due to decomposition of the second treatment gas or the gaseous treatment compound is prevented, and defects such as disruption of cultured cells due to the by-products do not occur. The inert gas is not particularly limited, and examples thereof include nitrogen gas, and the like.

In the present embodiment, a cleaning treatment may be performed after the surface modification treatment, the first surface-modifying group introduction treatment, the second surface-modifying group introduction treatment, and the post-treatment. For example, when the cleaning agent is water, a part of fluorine atoms (fluorine groups) directly chemically bonded to carbon atoms or the like is reacted with water molecules by cleaning, and a -OH group, a -COOH group, or the like can be directly chemically bonded to the carbon atoms or the like in place of the fluorine group.

As a result, the surface of the cell culture member 10 can be further hydrophilized. In addition, the first treatment gas, the second treatment gas, the treatment compound, and the byproduct that are not immobilized on the surface of the cell culture member 10 can be removed. The cleaning agent to be used is not particularly limited, and examples thereof include ethanol, isopropyl alcohol, water (for example, ultrapure water), toluene, and acetone. The cleaning conditions are not particularly limited, and the cleaning is usually performed at a cleaning temperature (temperature of the cleaning agent) of 0 °C to 100 °C for a cleaning time of 1 second to 60 minutes.

After the cleaning treatment, it is preferable to perform a drying treatment. The drying method is not particularly limited, and examples thereof include natural drying and drying by blowing nitrogen gas or the like. The drying conditions are not particularly limited, and the drying is usually performed at a drying temperature (when nitrogen gas or the like is blown, the temperature of the nitrogen gas) of 0 °C to 100 °C for a drying time of 1 second to 24 hours.

As described above, in the method for modifying the surface of the cell culture member 10 according to the present embodiment, it is possible to perform the surface modification to improve the adhesiveness to the adherent cell in the holding region by bringing the first treatment gas containing a gas containing a fluorine atom into contact with the holding region that holds the adherent cell. As a result, it is possible to provide the cell culture member 10 having excellent cell culture performance and excellent long-term stability.

### EXAMPLES

Hereinafter, preferred examples of the present invention will be exemplarily described in detail. However, the materials, blending amounts, conditions, and the like described in Examples are not intended to limit the scope of the present invention only to those unless otherwise specified.

### (Example 1)

First, a polystyrene microplate (trade name: IWAKI microplate for suspension culture 24well, manufactured by AGC Techno Glass Co., Ltd., hereinafter referred to as "microplate") was prepared, and the microplate was placed in a SUS316L chamber (capacity: 18 L).

Next, the inside of the chamber was replaced with nitrogen gas by vacuum replacement, and the temperature was raised at 4 °C/min under a stream of nitrogen gas (4 L/min) until the atmospheric temperature in the chamber reached 40 °C. Thereafter, the microplate was subjected to a thermostatic treatment for 1 hour.

Subsequently, the first treatment gas was introduced into the chamber, and the microplate was subjected to a surface modification treatment (fluorination treatment). The first treatment gas was introduced into the chamber until the pressure in the chamber reached atmospheric pressure by vacuum replacement. As the first treatment gas, a mixed gas containing: a fluorine gas having a concentration of 0.25 vol% with respect to the total volume of the first treatment gas; and a nitrogen gas was used. In the surface modification treatment, the chamber was sealed, the atmospheric temperature (treatment temperature) in the chamber was 40 °C, and the treatment time of the surface modification treatment was 17 minutes. Thereafter, the inside of the chamber was replaced with nitrogen gas by vacuum replacement, and cooled to room temperature under a stream of nitrogen gas (4 L/min).

Next, the microplate after the surface modification treatment was sufficiently cleaned with ultrapure water to perform a cleaning treatment, and then a nitrogen gas at 25 °C was blown to the microplate to perform a drying treatment. As a result, a microplate subjected to a surface modification treatment was produced as a cell culture member according to the present example. The temperature of the ultrapure water in the cleaning treatment was 25 °C, and the cleaning time was 5 minutes. The temperature of the nitrogen gas in the drying treatment was 25 °C, and the drying time was 8 hours.

### (Comparative Example 1)

In the present comparative example, a microplate (trade name: IWAKI microplate for suspension culture 24well, manufactured by AGC Techno Glass Co., Ltd.) of Example 1, which was not subjected to the surface modification treatment, was used.

### (Comparative Example 2)

In this comparative example, a microplate (trade name: IWAKI microplate for adherent culture 24well, manufactured by AGC Techno Glass Co., Ltd.) subjected to discharge treatment was used in place of the surface modification treatment of Example 1.

### (Elemental analysis)

Each of the microplates according to Example 1 and Comparative Examples 1 and 2 was subjected to elemental analysis. Elemental analysis was performed by X-ray photoelectron spectroscopy using a PHI 5000 VersaProbe III (trade name, manufactured by ULVAC-PHI, Inc.). The results are shown in Table 1.

### (Contact angle)

The contact angle of water was measured for each of the microplates according to Example 1 and Comparative Examples 1 and 2. The contact angle of water was measured with a contact angle meter (model number: DM-300, manufactured by Kyowa Interface Science Co., Ltd.). The results are shown in Table 1.

### (Measurement of binding energy of fluorine atom)

The microplate surface (surface subjected to fluorination treatment) according to Example 1 was analyzed by X-ray photoelectron spectroscopy (XPS) to measure the binding energy of the fluorine atom.

The measurement conditions of XPS were as follows. That is, an X-ray photoelectron spectrometer (model number: PHI VersaProbe III, manufactured by ULVAC-PHI, Inc.) was used, and an AlKα ray monochromatized with a monochromator was used as an X-ray to be emitted. The intensity in a range of binding energy of 679 eV to 699 eV was measured under the conditions of an X-ray output of 50 W, an acceleration voltage of 15 kV, a measurement region having a diameter of about 200 µmin one measurement, and a measurement interval of binding energy of 0.05 eV. As a result of the measurement, the binding energy of the fluorine atom was detected as a waveform having a peak value at 686.5 eV, as shown in FIG. 3. FIG. 3 is a graph showing the measurement result of XPS of the microplate according to Example 1.

### (Evaluation of cell culture)

Evaluation of cell culture was performed on each of the microplates according to Example 1 and Comparative Examples 1 and 2, using human embryonic kidney cells (HEK293T cells) and Syrian hamster kidney cells (BHK-21 (C-13) cells), respectively.

In the evaluation of cell culture, first, a culture solution was seeded on each of the microplates so that the number of cells was 1 × 10⁴ cells/well. The concentration of fetal bovine serum (FBS) in the culture solution was 10%.

Next, using this culture solution, human embryonic kidney cells and Syrian hamster kidney cells were cultured for 4 days under an environment of 37 °C and 5% CO₂. Thereafter, the medium was removed, the cells were detached, and the number of living cells of each cell was counted. The results are shown in Table 1.

### (Evaluation of cell culture in poor nutrient medium)

Evaluation of cell culture in a poor nutrient medium was performed on each of the microplates according to Example 1 and Comparative Examples 1 and 2, using Syrian hamster kidney cells (BHK cells).

That is, Syrian hamster kidney cells were cultured in the same manner as in the evaluation of cell culture described above except that a culture solution containing 5% fetal bovine serum (FBS) was used as a culture solution, and the number of living cells was counted. The results are shown in Table 1.

### (Evaluation of long-term stability of cell culture performance)

Next, evaluation of long-term stability of cell culture performance was performed on each of the microplates according to Example 1 and Comparative Examples 1 and 2, using human embryonic kidney cells (HEK293T cells).

For evaluation of long-term stability, first, a culture solution was seeded on each of the microplates so that the number of cells was 1 × 10⁴ cells/well. The concentration of fetal bovine serum (FBS) in the culture solution was 10%.

Next, this culture solution was stored at 25 °C for 30 days in the air. Thereafter, human embryonic kidney cells were cultured under an environment of 37 °C and 5% CO₂ for 4 days. After culture, the medium was removed, the cells were detached, and the number of living cells of human embryonic kidney cells was counted. The results are shown in Table 1.

**[Table 1]**

| | Elemental composition (at%) | | | Contact angle (°) | Evaluation of cell culture | | | |
|---|---|---|---|---|---|---|---|---|
| | | | | | Cell type | 10% FBS | | 5% FBS |
| | C | O | F | | | Evaluation results immediately after treatment (× 10⁴ cells) | Evaluation results after storage in air for 30 days (× 10⁴ cells) | Evaluation results immediately after treatment (× 10⁴ cells) |
| Example 1 | 75.0 | 11.0 | 14.0 | 71.4 | HEK293T | 31.7 | 31.5 | - |
| | | | | | BHK | 16.9 | - | 3.2 |
| Comparative Example 1 | 97.7 | 2.3 | 0.0 | 89.1 | HEK293T | 1.6 | 1.4 | - |
| | | | | | BHK | 0.9 | - | 0.2 |
| Comparative Example 2 | 86.7 | 13.3 | 0.0 | 72.3 | HEK293T | 31.2 | 23.4 | - |
| | | | | | BHK | 15.3 | - | 2.2 |

### (Evaluation of cell culture using primary cells)

Next, evaluation of cell culture was performed on each of the microplates of Example 1 and Comparative Examples 1 and 2, using mouse astrocyte cells.

In the evaluation of cell culture, first, the cerebral cortex was extracted from an ICR mouse fetus and enzymatically degraded, and the obtained mouse astrocyte cells were seeded on a microplate coated with laminin and polylysine. Thereafter, the mouse astrocyte cells were cultured for 15 days, and after completion of the cell culture was confirmed, the cells were detached.

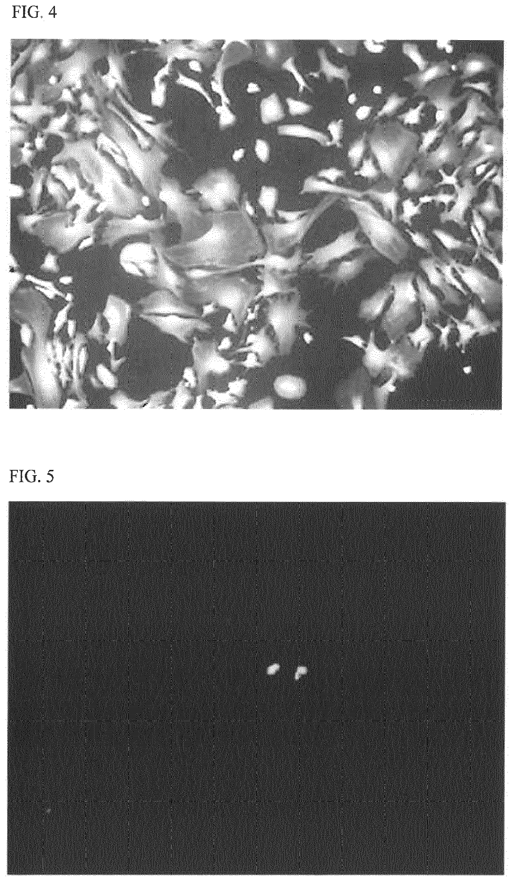

Subsequently, the detached cells were seeded again on each of the microplates so that the number of cells was 1 × 10⁵ cells/well. After 3 days, the mouse astrocyte cells in each of the microplates were fluorescently stained with calcein, and the state of the mouse astrocyte cells was confirmed with an inverted microscope. The results are shown in FIGS. 4 to 6. FIGS. 4 to 6 are views showing bright field images observed with an inverted microscope after mouse astrocyte cells are cultured using the microplates of Example 1 and Comparative Examples 1 and 2, respectively.

### Results

As a result of the elemental analysis by XPS, 14.0 at% of fluorine atoms were detected in the microplate after the fluorination treatment of Example 1. The result also reveals that the amount of the oxygen atom is increased as compared with the untreated microplate of Comparative Example 1. Further, in the microplate of Example 1, generation of the -OH group and the -COOH group as surface-modifying groups was confirmed from the peak positions of XPS. On the other hand, no fluorine atom was detected in the microplates of Comparative Examples 1 and 2.

As a result of the measurement of the contact angle of water, the contact angle of the microplate of Example 1 was smaller than that of Comparative Example 1 using an untreated microplate. This reveals that the microplate of Example 1 has improved wettability to water.

As a result of the evaluation of cell culture, the number of living cells was 31.7 × 10⁴ cells in the microplate of Example 1, and was 1.6 × 10⁴ cells in the untreated microplate of Comparative Example 1. This demonstrates that the microplate of Example 1 subjected to the surface modification treatment is superior in cell culture performance to the untreated microplate of Comparative Example 1.

In particular, in the evaluation of cell culture in a poor nutrient medium, the number of living cells was 3.2 × 10⁴ cells in the microplate of Example 1 subjected to the fluorination treatment, and this result demonstrates that the cell culture performance of the microplate of Example 1 is excellent as compared with those of the microplates of Comparative Examples 1 and 2.

Further, with respect to the long-term stability of cell culture performance, the number of living cells was 31.5 × 10⁴ cells in the microplate of Example 1 subjected to the fluorination treatment, and this result demonstrates that cell culture performance equivalent to that before storage in the air for 30 days is maintained. On the other hand, in the microplate of Example 1 subjected to plasma treatment, the number of living cells was 23.4 × 10⁴ cells, which was significantly reduced from the number of living cells before storage in the air for 30 days of 31.2 × 10⁴ cells, and this result demonstrates that the cell culture performance is reduced with the lapse of time.

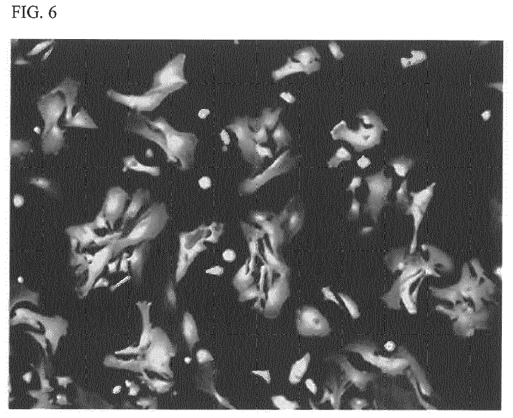

In the evaluation of cell culture using primary cells, in the microplate of Example 1 subjected to the fluorination treatment, it was confirmed that the neurites of the mouse astrocyte cells outgrew in a state in which the cells adhered to the microplate surface, as shown in FIG. 4. On the other hand, in the microplate of Comparative Example 1 not subjected to the surface modification treatment, the mouse astrocyte cells did not adhere to the microplate surface as shown in FIG. 5, and in the microplate of Comparative Example 2 subjected to the plasma treatment, the mouse astrocyte cells adhered to the microplate surface as shown in FIG. 6, but extension of the neurites of the cells was not sufficient as compared with that of Example 1. These results reveal that, also in the evaluation of cell culture using primary cells, the microplate of Example 1 is superior in cell culture performance to the microplates of Comparative Examples 1 and 2.

The above results reveal that the microplate after the fluorination treatment of Example 1 has better cell culture performance than that of the untreated microplate of Comparative Example 1. The above results also reveal that the microplate after the surface modification treatment of Example 1 maintains excellent cell culture performance even after storage in the air for 30 days, as compared with the microplate after the plasma treatment of Comparative Example 2.

### DESCRIPTION OF REFERENCE SIGNS

10: Cell culture member

## Claims

1. A cell culture member having at least a holding region that holds an adherent cell and contains a polymer compound, wherein at least a part of the holding region is a surface-modified region in which a fluorine atom is directly chemically bonded to a part of carbon atoms and/or silicon atoms constituting the polymer compound.

2. The cell culture member according to claim 1,
wherein a peak value of binding energy of the fluorine atom, as measured by X-ray photoelectron spectroscopy, is in a range of 680 eV to 690 eV.

3. The cell culture member according to claim 1 or 2,
wherein a surface-modifying group is directly chemically bonded to a part of other carbon atoms and/or other silicon atoms constituting the polymer compound in the surface-modified region,
the surface-modifying group is at least one selected from a group consisting of an -OR¹ group; a -COOR² group; a -COR³ group; a hydrocarbon group; a silyl group; a hydrocarbon group having at least one of a hetero atom, a halogen atom, or an unsaturated bond; a silyl group having at least one of a hetero atom, a halogen atom, or an unsaturated bond; a cyano group; a nitro group; a nitroso group; a phosphate group; a sulfonyl group; a thiol group; a thionyl group; and a halogen atom excluding a fluorine atom,
R¹ and R² are each independently a hydrogen atom; a metal atom; a hydrocarbon group; a silyl group; a hydrocarbon group having at least one of a hetero atom, a halogen atom, or an unsaturated bond; or a silyl group having at least one of a hetero atom, a halogen atom, or an unsaturated bond, and
R³ is a hydrocarbon group; a hydrocarbon group having at least one of a hetero atom or an unsaturated bond; or a silyl group having at least one of a hetero atom or an unsaturated bond.

4. The cell culture member according to any one of claims 1 to 3,
wherein the polymer compound is at least one polymer selected from a group consisting of polyvinyl chloride, polystyrene, polyethylene, polypropylene, polyvinyl acetate, polyurethane, cyclic polyolefin, polyether ether ketone, polyimide, polyamide imide, polycarbonate, polymethyl methacrylate, polyethylene terephthalate, acrylonitrile-butadiene-styrene, polyacrylonitrile, polyamide, polyvinyl alcohol, polyolefin, and a silicon-containing polymer compound.

5. A method for modifying a surface of a cell culture member having a holding region that holds an adherent cell and contains a polymer compound,
the method comprising a step of bringing a first treatment gas containing: a gas containing a fluorine atom; and an inert gas as an optional component into contact with at least a part of the holding region, and forming a surface-modified region in which the fluorine atom is directly chemically bonded to a part of carbon atoms and/or silicon atoms constituting the polymer compound.

6. The method for modifying a surface of a cell culture member according to claim 5, wherein a peak value of binding energy of the fluorine atom in the surface-modified region, as measured by X-ray photoelectron spectroscopy, is in a range of 680 eV to 690 eV.

7. The method for modifying a surface of a cell culture member according to claim 5 or 6,
wherein in the step, a surface-modifying group is directly chemically bonded to a part of other carbon atoms and/or other silicon atoms constituting the polymer compound by using a gas further containing a gas containing an oxygen atom as the first treatment gas,
the surface-modifying group is at least one selected from a group consisting of an -OR¹ group; a -COOR² group; a -COR³ group; a hydrocarbon group; a silyl group; a hydrocarbon group having at least one of a hetero atom, a halogen atom, or an unsaturated bond; a silyl group having at least one of a hetero atom, a halogen atom, or an unsaturated bond; a nitro group; a nitroso group; a phosphate group; a sulfonyl group; a thionyl group; and a halogen atom excluding a fluorine atom,
R¹ and R² are each independently a hydrogen atom; a metal atom; a hydrocarbon group; a silyl group; a hydrocarbon group having at least one of a hetero atom, a halogen atom, or an unsaturated bond; or a silyl group having at least one of a hetero atom, a halogen atom, or an unsaturated bond, and
R³ is a hydrocarbon group; a hydrocarbon group having at least one of a hetero atom or an unsaturated bond; or a silyl group having at least one of a hetero atom or an unsaturated bond.

8. The method for modifying a surface of a cell culture member according to claim 5 or 6,
further comprising
a step of bringing a second treatment gas containing another gas containing an oxygen atom into contact with at least a part of the holding region which has been brought into contact with the first treatment gas, and causing a surface-modifying group to be directly chemically bonded to a part of other carbon atoms and/or other silicon atoms constituting the polymer compound,
wherein
the surface-modifying group is at least one selected from a group consisting of an -OR¹ group; a -COOR² group; a -COR³ group; a hydrocarbon group; a silyl group; a hydrocarbon group having at least one of a hetero atom, a halogen atom, or an unsaturated bond; a silyl group having at least one of a hetero atom, a halogen atom, or an unsaturated bond; a nitro group; a nitroso group; a phosphate group; a sulfonyl group; a thionyl group; and a halogen atom excluding a fluorine atom,
R¹ and R² are each independently a hydrogen atom; a metal atom; a hydrocarbon group; a silyl group; a hydrocarbon group having at least one of a hetero atom, a halogen atom, or an unsaturated bond; or a silyl group having at least one of a hetero atom, a halogen atom, or an unsaturated bond, and
R³ is a hydrocarbon group; a hydrocarbon group having at least one of a hetero atom or an unsaturated bond; or a silyl group having at least one of a hetero atom or an unsaturated bond.

9. The method for modifying a surface of a cell culture member
according to claim 7 or 8,
further comprising
a step of bringing the fluorine atom directly chemically bonded to the part of the carbon atoms and/or the silicon atoms and/or the surface-modifying group directly chemically bonded to the part of the other carbon atoms and/or the other silicon atoms into contact with a treatment compound that reacts with the fluorine atom and/or the surface-modifying group, and substituting the fluorine atom and/or the surface-modifying group with a surface-modifying group derived from the treatment compound.

10. The method for modifying a surface of a cell culture member according to any one of claims 5 to 9,
wherein the polymer compound is at least one polymer selected from a group consisting of polyvinyl chloride, polystyrene, polyethylene, polypropylene, polyvinyl acetate, polyurethane, cyclic polyolefin, polyether ether ketone, polyimide, polyamide imide, polycarbonate, polymethyl methacrylate, polyethylene terephthalate, acrylonitrile-butadiene-styrene, polyacrylonitrile, polyamide, polyvinyl alcohol, polyolefin, and a silicon-containing polymer compound.
